# EUROPEAN PATENT SPECIFICATION

(11) **EP 3 135 227 B1**
(45) Date of publication and mention of the grant of the patent: **23.03.2022**
(21) Application number: 16185864.2
(22) Date of filing: 26.08.2016
(51) Int. Cl.: A61B 17/28, A61B 17/29, A61B 17/32

(54) **DISSECTING SURGICAL JAWS**
CHIRURGISCHE DISSEKTIONSBACKEN
MÂCHOIRES CHIRURGICALES POUR LA DISSECTION

(30) Priority: 26.08.2015 US 201514836244
(43) Date of publication of application: 01.03.2017
(73) Proprietor: Ethicon LLC, Guaynabo 00969 (PR)
(72) Inventor: HESS, Christopher J., Cincinnati, OH 45242 (US); SHELTON, IV, Frederick E., Cincinnati, OH 45242 (US); SAVAGE, Jeffrey L., Cincinnati, OH 45242 (US); GATES, Craig T., Cincinnati, OH 45242 (US); WITHERS, Douglas E., Cincinnati, OH 45242 (US)
(74) Representative: Carpmaels & Ransford LLP

(56) References cited:
- EP-A1- 0 584 787
- EP-A1- 0 835 637
- WO-A1-2006/041317
- DE-A1- 19 926 710
- GB-A- 2 360 481
- JP-U- S63 158 314
- US-A- 5 658 307
- US-A- 5 713 919
- US-A- 5 797 958
- US-A- 5 893 878
- US-A1- 2007 135 686
- US-A1- 2011 087 267

## Description

### FIELD

The present disclosure relates to surgical devices that can be used in tissue dissection, and more particularly to jaw members that can be used to perform multiple modes of dissection.

### BACKGROUND

Surgical devices are used in various open, endoscopic, and laparoscopic surgeries to grasp tissue volumes and blood vessels. The devices generally include a proximal housing, sometimes referred to as a handle portion when it is designed such that an operator grasps the device to operate it, a shaft extending distally from the proximal housing, and an end effector located at the distal end of the shaft. One or more actuators of the proximal housing can be used to operate the end effector. Devices used to grasp tissue volumes and blood vessels, sometimes referred to as dissectors, can include end effectors that have jaws for grasping the tissue and vessels disposed therebetween.

Current dissectors are designed such that different end effectors are used to perform different functions. For example, a person skilled in the art will recognize three different actions that are commonly performed during tissue dissection include: (1) dragging or abrasion formation; (2) clamping, pulling, or otherwise grasping; and (3) spreading to create or increase the size of an otomy. Many end effectors are specifically designed to perform one of these functions, or sometimes they are capable of performing two of these functions. However, existing end effectors are not typically designed in a manner that makes the end effectors effective to perform all three of these functions. For example, some jaws are designed to have interior features, such as teeth, to assist in firmly holding tissue therebetween. Such jaws are typically formed in a manner such that they taper to become smaller at a distal end, like a needlenosed pliers, so that they can be used with precision. Such a configuration is not typically useful when trying to perform functions like dragging or abrasion and/or spreading to create some types of otomies. As a result, during the course of a surgical procedure, a user typically switches the end effector at least once, and often more than once, to use a specific type of end effector to perform a specific function. This switching can be tedious, time consuming, and inconvenient, and can lead to errors in which one end effector is accidentally used in place of another with the two end effectors being designed for completely different purposes. The result can be mistakes, such as undesirably damaging tissue and the like.

Another issue with current end effectors exists with the way the formation of an otomy is performed. There is often very little control over the spreading function, which can lead to the size of an otomy increasing more rapidly than intended by a surgeon. This can lead to undesirable damage to tissue, such as creating a larger otomy than necessary.
GB 2360481A relates to forceps for cutting free the tissue in body cavities, in particular myomas sitting intramurally in the uterus, comprising a forceps jaw with two branches having gripping surfaces which are pivotable about a common axis. The branches are provided on their back outer surface with a tooth-like back profile over a part of their length. The back profile may have a saw-tooth profile, with teeth running transversely over the backs of the branches.

Therefore, it is desirable for end effectors to be designed that are able to be used across multiple functions, and in particular all three common, previously-identified functions commonly performed during dissection: (1) dragging or abrasion formation; (2) clamping, pulling, or otherwise grasping; and (3) spreading to create or increase the size of an otomy. It is also desirable for some of these functions, such as spreading to create an otomy, to be better controlled, thereby reducing the possibility of damaging tissue that was not intended to be treated.

### SUMMARY

The invention is defined by independent claim 1 and further embodiments are defined in the dependent claims. Dissector heads having improved features that enable dissection in three different modes are described. As a result, a single end effector can pierce, puncture, cut, spread, clamp, hold, drag, and/or cause to have abrasions formed on tissue, among other actions that can be performed by the end effector on the tissue. The features provided for to enable the three different dissection modes include features related to the geometry of the jaw assemblies. For example, the jaw(s) can have a bulbous distal tip configuration in which a width and/or thickness at a distal tip of the jaw(s) is greater than a width and/or thickness at an intermediate portion of the same jaw(s). Further, the jaw(s) can have particular curved configurations that are useful for performing particular dissection modes. According to the invention, ridges are formed on both the inner, tissue-facing surfaces and the outer surfaces of the jaws to assist in performing particular dissection modes.

Disclosures are also provided that relate to a three jaw dissector. The three jaw dissector can be arranged such that two of the three jaws are one length and the third is shorter. This allows the user to use the tips of the two longer jaws for otomy creation and fine dissection while still maintaining the grasping capabilities of the three jaw arrangement. This can be further enhanced by changing the timing of the motion of the three jaws. As the trigger is retracted from its distal most position, the two jaws move open while the third jaw is still stationary. In some embodiments, at about one-third of the opening trigger stroke, the third jaw can begin to move at a faster rate than the other two so that when fully open, all three jaws are fully opened without additional pressure being put on the tissue by the first two jaws to allow the third jaw to catch-up. In some instances, a three jaw dissector can be operated in a manner akin to a two jaw dissector, including by incorporating the disclosures pertaining to dissectors capable of operating in three different modes that are provided for herein.

In one aspect, jaws for a surgical device are described. The surgical device includes a first jaw opposed to and pivotally connected to a second jaw, with each jaw having a tissue-facing surface, an outer surface, a length that is greater than its width, and a thickness that is part of the outer surface. The tissue-facing surface of the second jaw faces the tissue-facing surface of the first jaw. The tissue-facing surfaces of the first and second jaws each also include a plurality of ridges that extend along a substantial portion of the width of the respective jaws, while the outer surfaces of the first and second jaws each include a plurality of ridges that extend along a substantial portion of the width of the respective jaws. The plurality of ridges of the outer surface of the jaws extend onto a portion of the outer surface that defines the thickness for that jaw.

In another aspect an endoscopic device is described. The endoscopic device includes an actuator and a jaw assembly. The jaw assembly is operably coupled to the actuator such that operation of the actuator can be configured to open and close opposed jaws of the jaw assembly. The jaw assembly can have a length, a width, and a thickness, with the length being longer than the width and the thickness being defined by a distance between top surfaces of the opposed jaws. The width of a distal end of the jaw assembly can be greater than a width of an intermediate portion along the length of the jaw assembly.

In yet another aspect, an exemplary surgical method not forming part of the invention is described. The surgical method includes performing at least one of the following steps (and sometimes both steps) in addition to dragging an outer surface of a jaw assembly against tissue to form abrasions in the tissue: (1) grasping tissue between first and second jaws of the jaw assembly; and (2) moving first and second jaws of the jaw assembly with respect to each other to spread tissue apart. The jaw assembly used to perform either or both of the grasping and moving steps is the same jaw assembly used to drag against tissue.

In other examples, any of the aspects above, or any apparatus or exemplary method not forming part of the invention described herein, can include one or more of the following features.

One or more ridges of the first jaw can be aligned with one or more ridges of the second jaw such that, in a closed configuration, the aligned one or more ridges of the first and second jaws can form one or more continuous ridges across the two jaws. A closed configuration can include when the internal surfaces of the jaws are in contact with one another, adjacent to each other, or would otherwise be in contact with one another or adjacent to each other if an object such as tissue was not disposed therebetween. In some embodiments, a portion of the outer surface of the first or second jaw that is opposed to the portion of the outer surface that defines the thickness for that jaw on which the plurality of ridges extend can be configured such that the plurality of ridges are not formed on that portion of the outer surface. The thickness of the first jaw at a distal end of the first jaw can be greater than a thickness of the first jaw at an intermediate portion along the length of the first jaw. The first jaw can have a distal end that is bulbous.

The first side surface of the first jaw can have a radius of curvature that is different from a radius of curvature of an opposed second side surface of the first jaw when the jaw is viewed from a top view, looking directly onto the outer surface of the first jaw from above. The first and second side surfaces can be a part of the outer surface of the first jaw. In some embodiments, the radius of curvature of the first side surface can be substantially the same along a majority of the length of the first jaw and the radius of curvature of the second side surface can change along the length of the second jaw. In some other embodiments, the distal end of the second side surface can be convex and an intermediate portion of the second side surface can be concave. In some embodiments, the width of the distal end of the first jaw can be greater than the width at an intermediate portion along the length of the first jaw.

A distal end of one of the first and second jaws according to the invention includes a tissue-grasping groove formed in the tissue-facing surface. The groove is substantially perpendicular to the plurality of ridges of the tissue-facing surface. A distal end of the other of the first and second jaws includes tissue-grasping tooth disposed on the tissue-facing surface. The tooth is substantially perpendicular to the plurality of ridges of the tissue-facing surface and is complimentary to the tissue-grasping groove of the other jaw.

A width at a proximal end of the jaw assembly can be greater than a width at the intermediate portion along the length of the jaw assembly. A thickness of the distal end of the jaw assembly can be greater than a thickness at the intermediate portion along the length of the jaw assembly. The plurality of ridges are formed on outer surfaces of each of the opposed jaws of the jaw assembly. In some embodiments, when viewing the jaw assembly from a top view, looking directly onto the top surface of one of the jaws of the jaw assembly, a first side surface of the distal end of the jaw assembly can be concave while an opposed second side surface of the distal end of the jaw assembly can be convex.

An outer surface of at least one of the first and second jaws includes a plurality of ridges formed on it and the exemplary surgical method not forming part of the invention can further include identifying and then using a particular portion of the outer surface of at least one of the jaws to be used in performing either the dragging step or the moving step. The identified particular region can include a distal tip of at least one of the first and second jaws, the distal tip having a thickness and a width that is greater than a thickness and a width of an intermediate portion of the same jaw, respectively. A portion of an outer edge of at least one of the first and second jaws can have multiple radii of curvature such that the identified particular region can be identified based on the radius of curvature at a particular location along the outer edge. An outer surface of at least one of the first and second jaws can include a plurality of ridges formed on it, the ridges extending along a substantial portion of a width of the at least one of the first and second jaws, and the ridges can extend further onto a side portion of the outer surface.

### BRIEF DESCRIPTION OF DRAWINGS

This disclosure will be more fully understood from the following detailed description taken in conjunction with the accompanying drawings, in which:
FIG. 1 is a side view of a modular surgical device having an elongate shaft with a distal mating feature that can couple to an end effector;
FIG. 2A is a side view of the distal mating feature of the elongate shaft of FIG. 1;
FIG. 2B is a side view of the distal mating feature of FIG. 2A having an obturator extending distally therethrough;
FIG. 3A is a perspective, cross-sectional view of a distal end of the elongate shaft of the device of FIG. 1A coupled to an end effector;
FIG. 3B is a perspective, cross-sectional view of the distal end of the elongate shaft and end effector of FIG. 3A with the end effector in a pushed-off configuration;
FIG. 4A is a partial perspective view of one end effector according to the invention that can be used with the surgical device shown in FIG. 1, showing a jaw assembly of the end effector in an open configuration;
FIG. 4B is side view of one jaw of the jaw assembly shown in FIG. 4A;
FIG. 4C is a top perspective view of the jaw shown in FIG. 4B;
FIG. 5A is a side view of another exemplary end-effector that can be used with the surgical device shown in FIG. 1 showing a jaw assembly that includes three jaws, the jaws being in an open configuration;
FIG. 5B is a side view of the jaw assembly shown in FIG. 5A in a closed configuration;
FIG. 5C is a side view of the jaw assembly shown in FIG. 5A in a partially open configuration;
FIG. 6A is a perspective view of a distal end of yet another exemplary end effector that can be used with the surgical device shown in FIG. 1, showing a jaw assembly that includes three jaws in a closed configuration;
FIG. 6B is a perspective view of the jaw assembly shown in FIG. 6A in a partially open configuration; and
FIG. 6C is a perspective view of the jaw assembly shown in FIG. 6A in an open configuration.

### DETAILED DESCRIPTION

Certain exemplary embodiments will now be described to provide an overall understanding of the principles of the structure, function, manufacture, and use of the devices and exemplary methods disclosed herein. One or more examples of these embodiments are illustrated in the accompanying drawings. Those skilled in the art will understand that the devices and exemplary methods not forming part of the invention specifically described herein and illustrated in the accompanying drawings are non-limiting exemplary disclosures and that the scope of the present invention is defined solely by the claims. The features illustrated or described in connection with one exemplary embodiment may be combined with the features of other embodiments.

In the present disclosure, like-numbered components of the various embodiments generally have similar features when those components are of a similar nature and/or serve a similar purpose. Further, to the extent features or sides of a structure are described herein as being a "first feature" or "first side" or a "second feature" or "second side," such numerical ordering is generally arbitrary, and thus such numbering can be interchangeable. Likewise, steps identified as a "first step" or a 'second step" are not necessarily required to occur in a particular order.

The terms "proximal" and "distal" are used herein with reference to a clinician manipulating the handle portion of the surgical instrument. The term "proximal" referring to the portion closest to the clinician and the term "distal" referring to the portion located away from the clinician. It will be further appreciated that, for convenience and clarity, spatial terms such as "vertical," "horizontal," "up," and "down" may be used herein with respect to the drawings. However, surgical instruments are used in many orientations and positions, and these terms are not intended to be limiting and/or absolute.

The present disclosure generally relates to surgical devices and exemplary methods not forming part of the invention for grasping tissue, blood vessels, or other objects in the body, collectively referred to herein as "tissue." Use of the word "tissue," however, by no means limits the type of objects that can be grasped using the devices and methods provided for in the present disclosure. A person skilled in the art will recognize many "non-tissue" objects that can be grasped in view of the present disclosure. More specifically, the surgical device can include jaws having features that aid in various modes of tissue dissection. These dissection modes include: (1) dragging or forming abrasions in tissue; (2) clamping, pulling, or otherwise grasping tissue; and (3) spreading jaws to create or increase a size of an otomy. As explained in great detail below, the features include but are not limited to forming teeth or grooves on portions of the outer surfaces of the jaws, as well as the size, shape, and overall configuration of the jaws. For example, in some instances the jaw(s) are wider and/or thicker at a distal end as compared to an intermediate portion of the same jaw(s). By way of further example, the ability to effectively perform multiple modes of dissection is aided by particular curvatures of portions of the jaws. The surgical device can also include three or more jaws, with some jaws being longer than others in some instances (e.g., in one illustrated embodiment having three jaws, two of the jaws are longer than the third). The three jaws can be configured such that the device operates in both a two jaw and a three jaw manner, *i.e.,* two of the jaws can be remain in contact such that the device operates in a two jaw configuration, or all three jaws can move away from each other such that the device operates in a three jaw configuration.

The various jaws provided for in the present disclosure can be operated using a variety of different surgical devices. Generally any jaws provided for herein can be an end effector that is coupled to a distal end of a surgical device and operated by the surgical device. The jaws can be fixedly mated to the surgical device, while in other examples they can be removably and replaceably mated to the device, which in turn provides the ability to use different types of end effectors and/or more easily clean the end effectors and the surgical device in between uses.

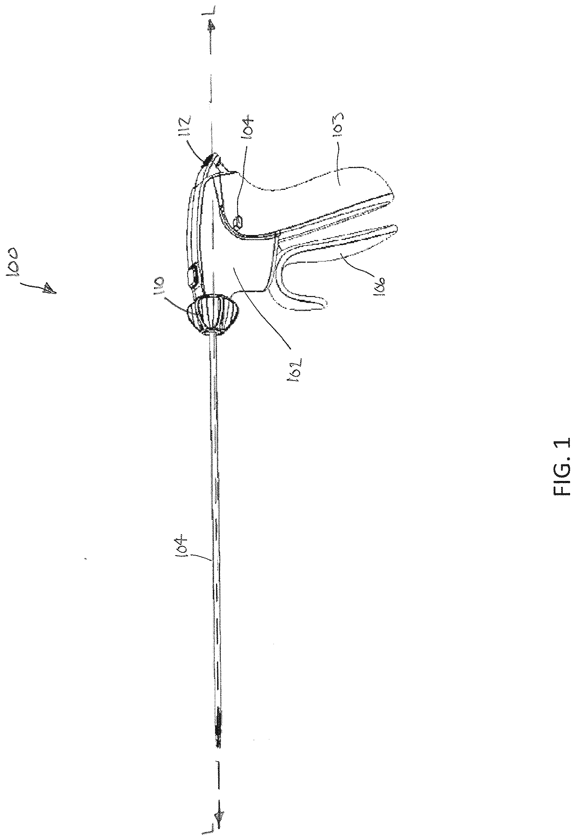

FIG. 1 shows an exemplary instrument or device 100 that can be used in conjunction with the jaws provided for in the present disclosure. As shown, the device 100 has a housing or handle portion 102 and an outer elongate shaft 104 extending distally from the housing 102, the shaft 104 being configured to have an end effector (e.g., jaws, not shown in FIG. 1) selectively coupled to it. The elongate shaft 104 can extend from a distal, upper portion of the housing 102, and it can be removably and replaceably attached to operable components in the housing 102 that are known to those skilled in the art. The housing 102 can include a stationary arm 103 and a closure actuator 106, such as a pivotable trigger, that is configured to move relative to the housing 102 to actuate an end effector when an end effector is coupled to the shaft 104. As shown, the closure actuator 106 can be coupled to a distal, lower portion of the housing 102, and when the end effector is any of the jaw assemblies provided for in the present disclosure, or jaw assemblies otherwise derivable therefrom, the closure actuator 106 can be operated to open and close the jaws.

The internal actuation components that can be used to translate motion of the closure actuator 106 to movement of jaws can have many different configurations, including being mechanically, electrically, and/or optically-based, and components of this nature are known to those skilled in the art, thus exact details about every such component is unnecessary. Some non-limiting examples of such components are discussed in greater detail in U.S. Application No. 14/836,069 (Atty. Dkt. No. 100873-716 (END7718USNP)), filed on August 26, 2015, and entitled "Surgical Device having Actuator Biasing and Locking Features," published as US 2017/0055970 A1. In general, such components can be disposed in, or attached to, portions of the housing 102 and/or the shaft 104. Some exemplary, non-limiting examples of these components include but are not limited to motors, controllers, and levers. Other implementations that can be used to actuate the jaws include but are not limited to actuator, gears, levers, triggers, and sliders. Further, a person skilled in the art will recognize other functions that the closure actuator 106 can perform, or other means of actuation.

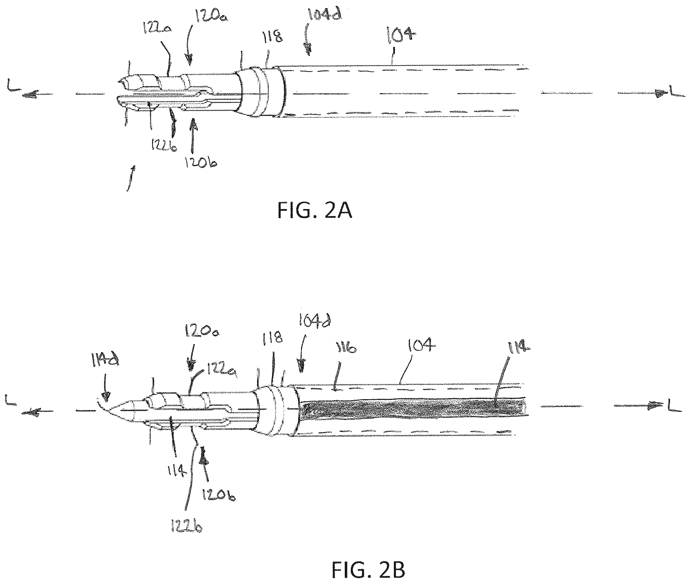

Still further, some non-limiting examples of features that can be incorporated as part of the device 100 include a locking switch 108 to selectively lock the closure actuator 106 in a fixed angular position relative to the housing, a knob 110 configured to rotate the elongate shaft 104, and thus an end effector such as jaw assemblies coupled thereto, and a locking member 112 configured to advance an inner shaft 114 (shown FIG. 2B) distally and proximally along a longitudinal axis L of the shaft 104. As explained below, movement of the inner shaft 114, as well as an intermediate shaft 116 (shown in FIGS. 2A and 2B), can help couple and decouple an end effector from a distal end 104d of the elongate shaft 104, as well as to operate the end effector via the closure actuator 106 when the end effector is coupled to the device 100.

FIGS. 2A and 2B illustrate the distal end 104d of the shaft 104 in greater detail, including exemplary attachment mechanisms located at the distal end 104d of the elongate shaft 104 so that an end effector like the jaws provided for herein (not shown in FIGS. 2A and 2B) can be mated to the shaft 104. While the attachment mechanism can vary, in the illustrated embodiment a circumferential groove 118 can be positioned around an outer surface of a distal portion of the shaft 104. First and second arms 120a, 120b can project distally from the distal end 104d of the shaft 104 and can be coupled to or otherwise integrally formed on the intermediate shaft 116. The arms 120a, 120b can be axially slidable relative the elongate shaft 104 and can be resiliently deflectable medially into the gap. The arms 120a, 120b can each have a mating feature, which in this shaft is a stepped lateral notch 122a, 122b.

A distal tip 114d of the inner shaft 114 (shown as a shaded region in FIG. 2B) can be positioned medially relative to the arms 120a, 120b and can be axially slidable relative to the arms 120a, 120b. More specifically, the distal tip 114d of the inner shaft 114 can slide between an unlocked position in which the distal tip 114d of the inner shaft 114 is proximal to the arms 120a, 120b, allowing medial deflection of the arms 120a, 120b (as shown in FIG. 2A), and a locked position in which the distal tip 114d of the inner shaft 114 is aligned with or distal to the arms 120a, 120b, thereby preventing medial deflection of the arms 120a, 120b (as shown in FIG. 2B). In certain aspects, the inner shaft 114 and the arms 120a, 120b can slide independently along the longitudinal axis L of the elongate shaft 104. As shown in FIG. 2B, the distal tip 114d of the inner shaft 114, which is also referred to herein as an obturator tip, can be pointed and/or sharpened such that the distal tip 116d can pierce through tissue. In the illustrated embodiment, the distal ends of the arms 120a, 120b and the distal end 104d of the elongate shaft 104 can taper from a proximal-to-distal direction and this can facilitate passing the arms 120a, 120b and the elongate shaft 104 through an incision (not shown), such as an incision formed by the distal tip 114d. As will be appreciated by persons skilled in the art, the distal tip 114d of the inner shaft 114 need not be sharpened or pointed and the outer and intermediate shafts can include various types of attachment mechanisms for mating with an end effector and need not include a taper, grooves, etc.

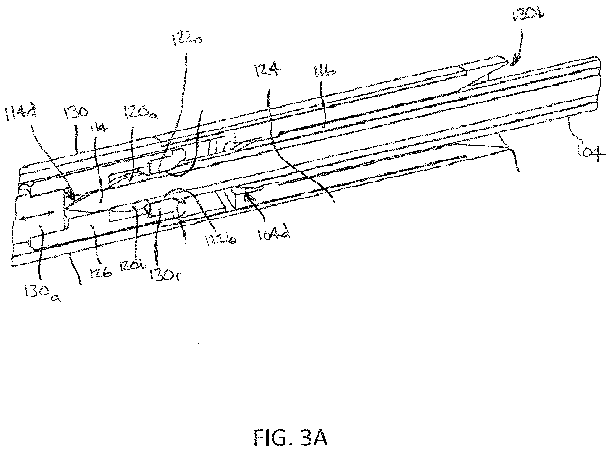

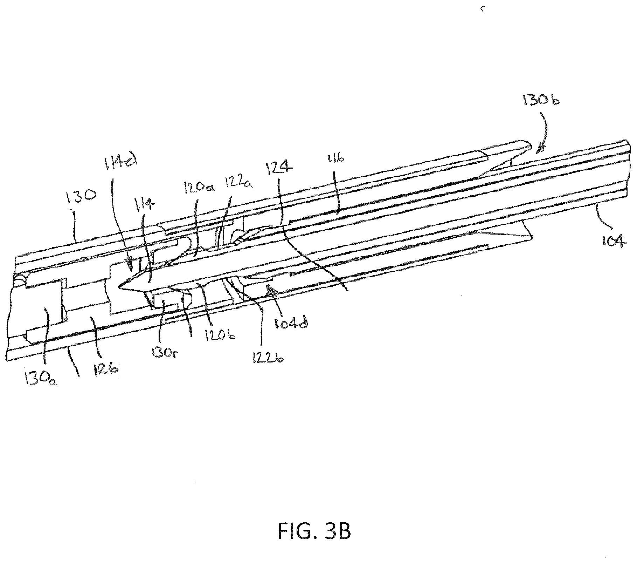

FIG. 3A illustrates an exemplary interaction between an end effector 130 (*e.g*., jaw assemblies provided for herein or otherwise derivable therefrom) and the intermediate and inner shafts 114, 116. As shown, the end effector 130 has a proximal end 130p and an opening 130b that extends over a distal end 104d of the elongate shaft 104. In the illustrated embodiment, the groove 118 of the arms 120a, 120b of the intermediate shaft 116 mates with a rib 124 of the end effector 130 preventing relative axial motion. The lateral grooves 122a, 122b of the arms 120a, 120b mate to a ring 130r of the end effector 130 preventing relative axial motion. Rib 124 is rigidly connected to an outer housing of the end effector 130, and the ring 130r is rigidly and fixedly connected to a jaw actuator 130a of the end effector 130 via a coupling 126. Accordingly, axial movement of the arms 120a, 120b relative the intermediate shaft 116 will cause axial movement of the jaw actuator 130a relative the housing 130h of the end effector 130, thereby causing the jaws to open and close. FIG. 3B illustrates the end effector 130 ready to be detached from the elongate shaft 104. In particular, distally advancing the arms 120a, 120b of the intermediate shaft 116 can push the ring 130r distally until the rib 124 unseats from the groove 118 and allows the distal end 104d of the elongate shaft 104 to be removed from the end effector 130.

A person skilled in the art will recognize that the surgical device 100 illustrated herein is just one of many different surgical devices and designs with which the present disclosures related to jaw assemblies can be used. The description of the same is for illustrative purposes to provide one way by which the jaws can be actuated. The description of the device 100 in no way limits the ability for the jaws described herein to be used in conjunction with many other devices and systems. Accordingly, by way of non-limiting example, while in the illustrated embodiment the end effector 130 is described as extending over the distal end 104d of the elongate shaft 104, in other instances, using any techniques known to those skilled in the art, the jaw assemblies of the present disclosures can be coupled directly to a distal tip of the elongate shaft 104, coupled in some fashion inside the distal end 104d of the elongate shaft 104, or coupled to a coupling, such as the coupling 126, which itself is coupled in some fashion to the elongate shaft 104. Any way by which the jaws of the present disclosure can be actuated is acceptable. Other examples of surgical devices that a person having ordinary skill in the art could use in conjunction with the present disclosures includes but are not limited to the devices provided for in U.S. Patent Application Publication No. 2011/0087267, entitled "Method for Exchanging End Effectors In Vivo,".

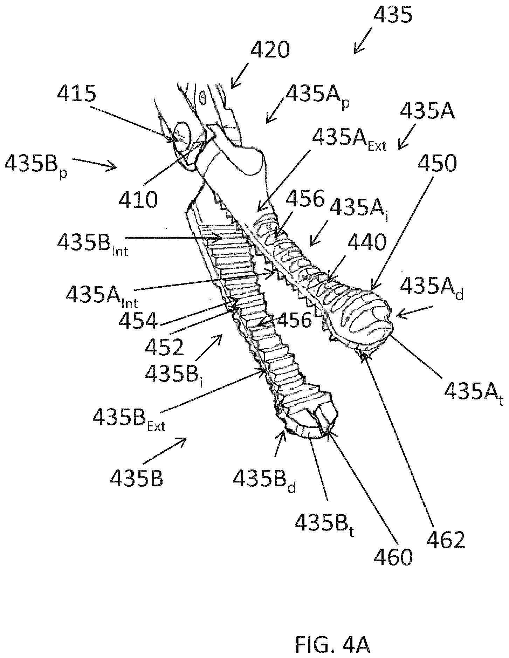

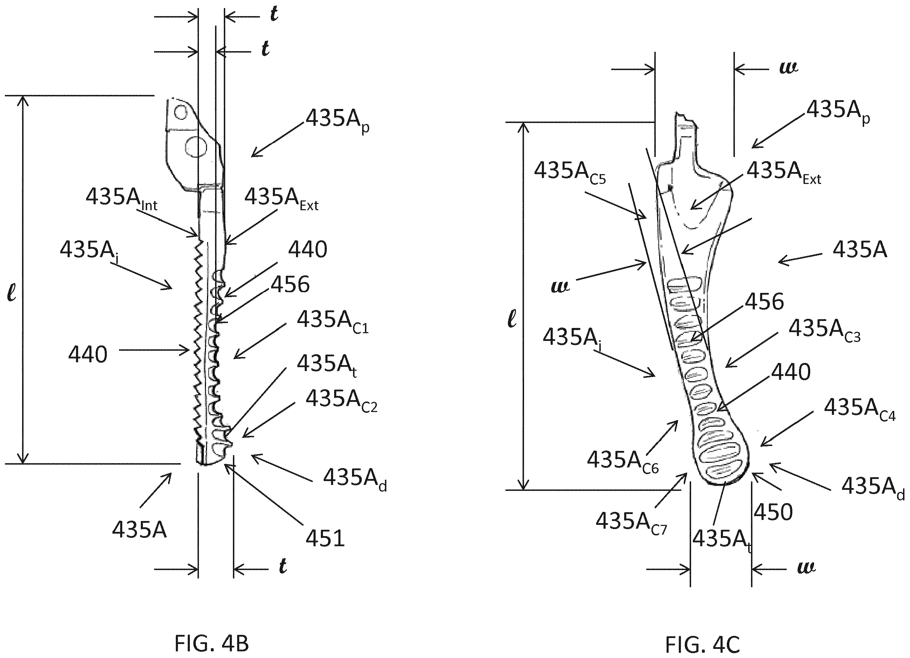

The present disclosure provides for jaw assemblies that can have a variety of sizes, shapes, and configurations and used in conjunction with a variety of different surgical instruments, devices, and systems. In FIGS. 4A-4C, the end effector is a jaw assembly 435 having two jaws according to the invention:
a first jaw 435A and a second jaw 435B. The jaws 435A, 435B are arranged such that they are pivotally connected to each other. The jaws 435A, 435B can also be arranged such that they can rotate with respect to one another to grasp or hold tissue disposed therebetween. The jaws 435A, 435B can be connected via a pivot pin 415 at a pivot point 410 located on a proximal end of the jaws 435Aₚ, 435Bₚ. As shown, the pivot pin 415 couples the jaws 435A, 435B to a substantially rigid clevis 420 that provides stability and control of the jaws 435A, 435B, thereby minimizing a condition in which the jaws operate in a floppy or flimsy manner. Each jaw 435A, 435B is a generally elongated member that has a length, *l*,
a width, w, and a thickness, *t*, as illustrated between FIGS. 4B and 4C. The length ℓ of each jaw can be greater than its respective width 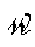 and its respective thickness *ℓ*. Further, both the width 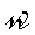 and the thickness *ℓ* for one or both jaws 435A, 435B can change along the length *ℓ* as illustrated and as described in greater detail below. Further, each jaw can be described as having internal or tissue-facing surfaces 435A_{Int}, 435B_{Int}, which are opposed and can generally be the portions configured to grasp tissue therebetween, and external surfaces 435A_{Ext}, 435B_{Ext}, which are the remainder of the surfaces of the jaws 435A, 435B that generally face externally, and thus can include top and side surfaces or portions as described herein.

The jaws 435A, 435B can include features configured to aid in performing the three previously described tissue dissection modes: (1) dragging or abrasion formation; (2) clamping, pulling, or otherwise grasping; and (3) spreading to create or increase a size of an otomy. One such feature provided for is curved portions of one or both jaws 435A, 435B, sometimes referred to as generally convex or generally concave portions. In the illustrated embodiment, the jaw 435A includes seven curved portions: a generally concave first curved portion 435Aci and a generally convex second curved portion 435A_{C2} of a top portion of the external surface 435A_{Ext} when viewed from a side as shown in FIG. 4B, a generally concave third curved portion 435A_{C3} and a generally convex fourth curved portion 435A_{C4} of an outer portion of the external surface 435A_{Ext} when viewed from the top, as shown in FIG. 4C, and a generally convex fifth curved portion 435Ac5, a generally concave sixth curved portion 435Ac6, and a generally convex seventh curved portion 435A_{C7} of an inner portion of the external surface 435A_{Ext} when viewed from the top, as also shown in FIG. 4C. The use of such curved portions in jaws can be beneficial because it provides different surfaces on the end effector that can be used to mimic the shape of the organs or tissues being treated.

The curved portions 435Aci, 435A_{C2}, 435A_{C3}, 435A_{C4}, 435Ac5, 435Ac6, and 435A_{C7} result in configurations in which the width 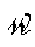 and thickness *ℓ* of the jaw 435A changes across the length ℓ of the jaw 435A. Accordingly, as shown in FIG. 4B, in view of the concavity of the curved portions 435A_{C1} and 435A_{C2}, the thickness ℓ at an intermediate portion 435Aᵢ of the jaw 435A is less than the thickness ℓ at a proximal end 435Aₚ and at a distal end 435A_{d} of the jaw 435A. In the illustrated embodiment, the thickness *ℓ* at the distal end 435A_{d} is also greater than at the proximal end 435Aₚ, with the thickness at the distal end 435A_{d} contributing to the formation of a bulbous distal region having a curved distal-most tip 435Aₜ. Likewise, as shown in FIG. 4C, in view of the concavity of the curved portions 435A_{C3}, 435A_{C4}, 435A_{C5}, 435A_{C6}, and 435A_{C7}, the width at the intermediate portion 435Aᵢ is less than the width at the proximal and distal ends 435Aₚ, 435A_{d}, with the thickness at the distal end 435A_{d} contributing to the formation of the aforementioned bulbous distal region having a curved distal-most tip 435Aₜ.

While the curved portions 435Aci, 435A_{C2}, 435A_{C3}, 435A_{C4}, 435Ac5, 435Ac6, and 435A_{C7} are described as being generally convex and generally concave, the curves associated with these portions can vary across the length *ℓ* of the jaw 435A, and thus there can be different radii of curvature for each curved portion 435Aci, 435A_{C2}, 435A_{C3}, 435A_{C4}, 435Ac5, 435Ac6, and 435A_{C7}. For example, a jaw surface can have various curved portions, each of which are curved at different curvatures. Further, the curved portion residing on a surface of a jaw can form one or more compound curves or compound angles. Each compound angle (or compound curve) can include more than one radius of curvature. In the illustrated embodiment, when viewed from the top, looking directly onto the outer surface of the jaw, as shown in FIG. 4C, a radius of curvature of a first side surface, also referred to as an outer surface *(i.e.,* the side surface in which the curved portions 435A_{C5}, 435A_{C6}, and 435A_{C7} are identified) is different from a radius of curvature of an opposed second side surface of the same jaw, also referred to as an inner surface *(i.e.,* the side surface in which the curved portions 435A_{C3} and 435A_{C4} are identified). More particularly, in the illustrated embodiment, the radius of curvature of the first side, inner surface is substantially the same along a majority of its length, while the radius of curvature of the second side, outer surface changes along the length of the second side surface.

The size, shape, and configuration of one jaw can, but does not necessarily have to, mirror the size, shape, and configuration of other jaws with which the one jaw is used. The size, shape, and configuration of each jaw can be independent or dependent of the others depending, at least in part, on the configuration of the other components with which the jaw(s) is being used, the type of procedure being performed, and the desired configuration of the user. Accordingly, although in the illustrated embodiment the curved portions are discussed with reference to the jaw 435A, the jaw 435B can likewise include curved portions that mimic those of the jaw 435A, or it can include flat or other curved portions as desired. Any of the lengths, widths, and thickness at any portion of the jaws can be the same or different, again depending, at least in part, on the configuration of the other components with which the jaw(s) is being used, the type of procedure being performed, and the desired configuration of the user. Further, the internal and external surfaces of the same jaw need not assume similar curvatures. For example, one of the internal or external surfaces may include one or more curved portions while the other does not include a curved portion. Still further, to the extent portions are called "curved portions," they do not necessarily have to be curved. Portions along a length of the jaws 435A, 435B may be un-curved or straight. Still further, while the illustrated portion identifies seven curved portions 435Aci, 435A_{C2}, 435A_{C3}, 435A_{C4}, 435Ac5, 435Ac6, and 435A_{C7}, any number of curved portions can be provided for without departing from the present disclosure, depending, at least in part, on the configuration of the anatomy with which the jaws 435A, 435B are being used and the type of procedure being performed. Any number and design of convex or concave portions can be provided for in the jaws 435A, 435B.

While the jaws 435A and 435B can have various configurations, a length *ℓ* of the jaws can be approximately in the range of about 10 millimeters to about 50 millimeters, a width 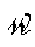 of the jaws can be approximately in the range of about 1 millimeter to about 10 millimeters, and a thickness *ℓ* of the jaws can be approximately in the range of about 1 millimeters to about 5 millimeters, with the width and thickness being able to have different values across the length of the respective jaw. Further, a length *ℓ* of the jaws 435A and 435B can be different across two surfaces, such as one surface (*e.g.,* the internal side surface in which the curved portions 435A_{C5}, 435A_{C6}, and 435A_{C7} are identified) being longer than an outer surface *(e.g.,* the external side surface in which the curved portions 435A_{C3} and 435AC4 are identified). In one exemplary embodiment, a length *ℓ* of the top jaw 435A and of the bottom jaw 435B is approximately 25 millimeters, a smallest width 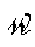 of the top jaw 435A and of the bottom jaw 435B, at intermediate portions 435Aᵢ and 435Bᵢ, respectively, is approximately 2 millimeters, a largest width 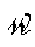 of the top jaw435A and of the bottom jaw 435B, at the respective bulbous distal tips 435Aₜ and 435Bₜ, is approximately 4 millimeters, a smallest thickness *ℓ* of the top jaw435A and of the bottom jaw 435B, at the respective intermediate portions 435Aᵢ and 435Bᵢ, is approximately 1 millimeters, and a largest thickness *ℓ* of the top jaw 435A and of the bottom jaw 435B, at the respective bulbous distal tips 435Aₜ and 435Bₜ, is approximately 2 millimeters.

The jaws can also be formed of a variety of materials, including more than one material, and such materials include but are not limited to surgical stainless steel, other 300 and 400 series stainless steels, titanium, and aluminum.

Another feature configured to aid in performing the three aforementioned dissection modes is the inclusion of non-uniform surfaces on both the internal surfaces 435A_{Int}, 435B_{Int} and the external surfaces 435A_{Ext}, 435B_{Ext} of the jaws 435A, 435B. As shown in FIGS. 4A, 4B, and 4C, the surfaces 435A_{Int}, 435B_{Int}, 435A_{Ext}, and 435B_{Ext} can include one or more serrations, teeth, or ridges 440 (hereinafter "ridges"), and corresponding grooves 456, the combination of which can be referred to as an undulating surface. The use of the term undulating does not necessarily mean that ridges 440 or grooves 456 have any sort of set pattern or consistency, though they can if desired. The inclusion of the ridges 440 on the internal surfaces 435A_{Int}, 435B_{Int} can assist at least in the dissection mode of clamping, pulling, or otherwise grasping tissue by increasing the friction between the tissue and the jaws 435A, 435B. The inclusion of the ridges 440 on the external surfaces 435A_{Ext}, 435B_{Ext} can assist at least in the dissection mode of dragging or abrasion formation by creating additional surfaces that can grip tissue to drag it or form abrasions on the tissue by contacting the tissue with the external surfaces 435A_{Ext}, 435B_{Ext}, and can also prevent tissue from sliding off the jaws 435A, 435B when spreading or dragging tissue. The ridges 440 on the external surfaces 435A_{Ext}, 435B_{Ext} can align along at least a portion of the length ℓ of the jaws 435A, 435B such that at least one ridge from one external surface extends to the other external surface to form a continuous ridge between the two jaws when the jaws are in a closed configuration, *i.e.,* a configuration in which the internal surfaces 435A_{Int}, 435B_{Int} of the jaws 435A, 435B are in contact with one another, adjacent to each other, or would otherwise be in contact with one another or adjacent to each other if an object such as tissue was not disposed therebetween. Further, including but not limited to embodiments in which ridges 440 on the external surfaces 435A_{Ext}, 435B_{Ext} are aligned along at least a portion of the length ℓ of the jaws 435A, 435B, the ridges 440 formed on the external surface 435A_{Ext} and/or 435B_{Ext} can align along at least a portion of the length ℓ of the respective jaw with the ridges 440 on the internal surface 435A_{Int} and/or 435B_{Int} such that the ridge from the external surface extends to the internal surface of that same jaw.

The ridges 440 and corresponding grooves 456 can be formed using a variety of techniques known to those skilled in the art. Notably, to the extent the present disclosure indicates that the ridges or grooves are "formed on" surfaces of the jaws, a person skilled in the art will recognize that other ways of providing ridges and grooves can also be used, such as by attaching a separate component having ridges and grooves formed it onto the jaws. The term "formed on" does not in any way limit the way by jaws can include grooves and ridges. The ridges 440 can be formed by overmolding, while in other examples a separate material (e.g., an elastomer or a metal) can be coupled to the external surface(s) 435A_{Ext}, 435B_{Ext} of the jaw(s) 435A, 435B. A variety of materials can be used to form the ridges 440, and the jaws 435A, 435B for that matter, including but not limited to thermoplastic elastomers, rubber, and silicone. When the ridges 440 are formed of materials that have some elasticity, the ridges 440 can flex and deform to help grip tissue without tearing it.

A location of the ridges 440 on either or both of the internal and external surfaces 435A_{Int}, 435B_{Int}, 435A_{Ext}, and 435B_{Ext} of the jaws 435A, 435B can vary, depending, at least in part, on the desired configuration of the user and the type of procedure being performed, and the location does not need to be uniform for each of the jaws 435A, 435B. Thus, ridges 440 can be formed on a substantial majority of the surface area of the jaws, while in other examples portions of either or both the internal and external surfaces 435A_{Int}, 435B_{Int}, 435A_{Ext}, and 435B_{Ext} can include smooth surfaces that do not have ridges 440 formed therein or thereon.

One or more ridges 440 and/or grooves 456 may extend along an entire top portion of the external surface 435A_{Ext} of the jaw 435A to at least one side portion of the external surface(s) 435A_{Ext}, such as an outer side portion of the external surface 435A_{Ext}, as shown in FIG. 4B. Such a configuration can also exist on the jaw 435B. The ridges 440 can extend to the opposed side surface, *e.g.,* the inner side portion of the external surface 435A_{Ext}, such that the ridges 440 extend along a curvature of the jaw 435A from the top surface (or bottom surface for jaw 435B) to the side surface, while in other examples the opposed side surface cannot include ridges formed thereon. The portion of the length ℓ along which the ridges 440 and grooves 456 extend can vary, from as little as just at the distal tip portion 435Aₜ to along a substantial majority of the length of the jaw 435A. Further, with respect to the internal surfaces 435A_{Int}, 435B_{Int}, the ridges 440 formed on the opposed internal surfaces 435A_{Int}, 435B_{Int} of the first and second jaws 435A, 435B can be complementary such that when the jaws 435A, 435B are closed together, the ridge 440 on the jaw 435A fits within the corresponding groove 456 on the opposing jaw 435B, thus providing for little or no space between the two jaws.

The ridges 440 can have any size or shape known to those skilled in the art. The sides of the ridges 440 can be generally rectangular in nature *(e.g.,* in the form of castellation) and/or curved (e.g., appear as crenulations). Each ridge 440 can have at least two sidewalls 452, 454 and the sidewalls of adjacent ridges can define the groove 456 between the sidewalls of the ridges. The area between the ridges *(i.e.,* the groove 456) can assume various shapes and geometries. For example, the groove can be rectangular, rounded, curved, semi-frustoconical, semi- cylindrical, semi-pyramid shaped, or can assume other shapes (as well as other compound shapes).

Further, the distance between the neighboring ridges 440 *(i.e.,* the width of the grooves 456, hereinafter groove width) can vary. For example, two neighboring ridges on a surface of a jaw can define a groove having a first general shape and a first groove width, while one of those ridges and another neighboring ridge can define a groove having a second, different, general shape and a second, different, groove width. The shape between ridges and grooves can be uniform across a portion of a length, or the entire length along which the ridges and grooves are formed. Each ridge 440 can be curved such that the distance between the adjacent ridges does not remain uniform along the length and width of the jaw surface. For example, the adjacent neighboring ridge sidewalls can be curved or can taper such that the groove width (distance between the two ridges sidewalls) varies along the length and width of the jaw surface.

Further, the ridges 440 can extend radially transverse to the longitudinal axis L of the shaft 104. Still further, one or more sidewalls of a ridge 440 and the surface of the jaw on which the ridge resides can form an angle. The angle formed between the ridge and the sidewall can be any angle, ranging from 0 degree to 180 degrees. The angle formed between one sidewall of a ridge and the jaw surface can be different from the angle formed between the other sidewalls of the ridge and the jaw surface. Similarly, the angles made between the sidewalls of one ridge and the jaw surface can vary from the angles formed between the other ridges and the same jaw surface and/or the angles made between other ridges and other jaw surfaces.

A height of the ridges 440, and thus the depth of the grooves 456, can by the same or different across the length and/or the width of the individual jaws 435A, 435B. The ridges 440 and grooves 456 can have heights approximately in the range of about 0.25 millimeters to about 2 millimeters, and in some examples one or more ridges, and sometimes a majority of the ridges, can have a height of approximately 0.5 millimeters. Further, the sidewalls of the same ridge 440 and/or the same groove 456, can have different heights. For example, a ridge 440 can have a sidewall(s) that is longer *(e.g.,* extends out further, when measured with respect to the jaw surface) than its other sidewalls (*e.g*., the top surface of the ridge can be tapered).

As noted previously, the ridges 440 can cover one or more small regions and/or an entire surface of a jaw. Further, one or more jaw surfaces can be arranged such that they do not include any ridges. The number of ridges disposed on each surface, their positioning, shape, orientation, geometrical configuration, depth, and/or their spacing relative to one another can vary depending on the application (*e.g*., the nature and elasticity of the tissue).

In the embodiment according to the invention shown in FIG. 4A, the jaws 435A, 435B are illustrated in an open configuration. The jaws 435A, 435B can move relative to one another across various orientations and/or positions. For example, the jaws 435A, 435B can fully close such that at least one spot on the internal surface 435A_{Int} of the first jaw 435A comes in direct contact with at least one spot on internal surface 435Bint of the second jaw 435B. Additionally or alternatively, the jaws can be partially opened or closed with respect to each other such that their internal surfaces 435A_{Int}, 435B_{Int} form angles ranging from zero to 360 degrees with each other.

As noted above, a jaw 435A, 435B can assume various curvatures and geometries and surfaces include ridges 440 in one or more locations. Accordingly, a thickened area, *e.g.,* the bulbous distal tip 435Aₜ, 435Bₜ can have curvatures (*e.g.,* an over compound angle) that are different from the curvatures of the surrounding areas. Additionally or alternatively, a thickened area can include one or more ridges 440. As noted, the one or more ridges 440 disposed on the jaw surface 435A_{Int}, 435B_{Int}, 435A_{Ext}, and 435B_{Ext} (and/or the thickened areas), extend along the surface of the jaw and/or around the diameter of the jaw surface. Accordingly, a thickened area can have one or more ridges 440, as described above, disposed thereon. The ridges 440 can extend along the diameter of the thickness and/or possibly to the opposing surface of the jaw and/or along any geometry that is disposed on the other side of the jaw.

The distal end of the tissue-facing surfaces 435A_{Int}, 435B_{Int} of the jaws 435A_{d}, 435Bd can also include one or more 462 ridges arranged for capturing or grasping tissue (hereinafter "grasping ridge"). Such grasping ridges can be disposed on one or more other sections of a jaw surface. Similar to the surface ridges 440 described above, a grasping ridge 462 can assume various shapes, orientations, geometrical configurations, and/or depths. For example, the grasping ridge 462 can be rectangular, rounded, curved, semi-frustoconical, semi- cylindrical, semi-pyramid shaped, or can assume other shapes (as well as other compound shapes). The grasping ridge 462 can be disposed anywhere on the distal end 435A_{d}, 435B_{d} of the internal surface 435A_{Int}, 435B_{Int} of the jaws 435A, 435B. In the illustrated embodiment, the grasping ridge 462 is formed on the tissue-facing surface 435A_{Int} of the jaw 435A, substantially perpendicular to the ridges 440 formed on the tissue-facing surface 435A_{Int}.

The distal end of the jaws includes one or more grasping grooves 460 arranged to mate with the one or more grasping ridges 462 positioned on the opposing jaw. For example, as shown in FIG. 4A, the grasping groove 460 is formed in the tissue-facing surface 435B_{Int} of the jaw 435B and is arranged to mate with the grasping ridge 462, and is thus substantially perpendicular to the ridges 440 formed on the tissue-facing surface 435B_{Int}. Similar to the surface grooves described above, the grasping grooves can assume various shapes, orientations, geometrical configurations, and/or depths, and can be located in any number of locations with respect to the jaw(s). Although shown and described as being arranged to receive a complimentary grasping ridge positioned on an opposing jaw, one or more grasping grooves can be disposed on a jaw surface without having any complimentary grasping ridges on the opposing jaw.

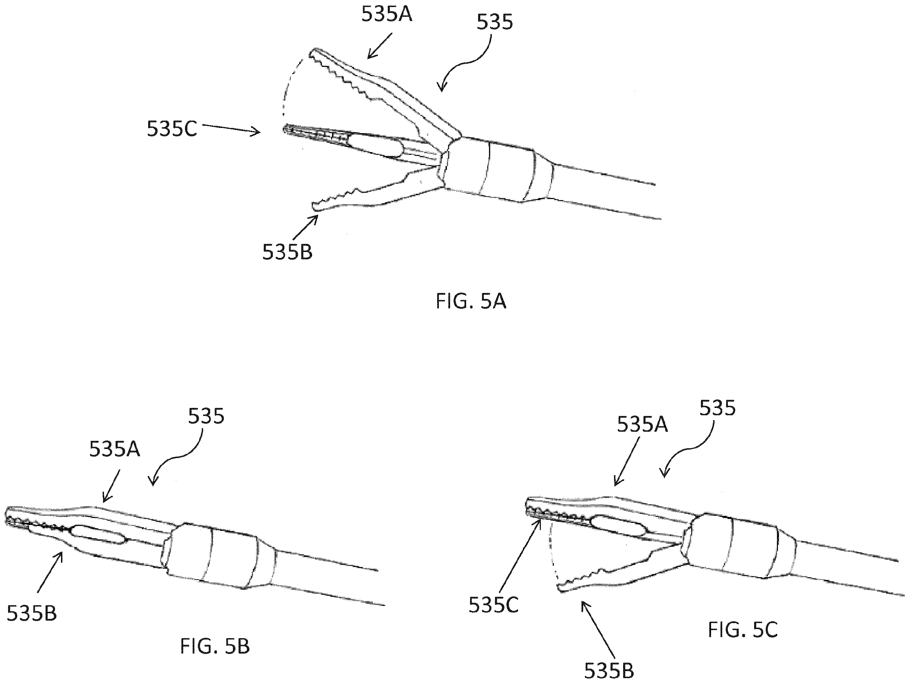

Although the medical device and the jaw assembly 435 shown in FIG. 4A has two jaws 435A, 435B, in other medical devices and the jaw assemblies, such as for the jaw assembly 535 illustrated in FIGS. 5A-5C and described in greater detail below, more than two jaws can be used. In view of the disclosures provided for herein, a single assembly having more than two jaws can be operated effectively to perform each of the three tissue dissection modes previously identified. Regardless of the number of jaws included in a jaw assembly 435, the jaw features described herein, such as width, length, thickness, geometry, curvatures, thickened areas, bulbous tip, and ridge arrangements, can be incorporated into any portion of any jaw of a jaw assembly without departing from the present disclosure.

Referring to FIG. 5A, the jaw assembly 535 includes three jaws 535A, 535B, 535C. As noted, each of the jaws 535A, 535B, 535C can have different lengths, widths, geometries, curvatures, thickened areas, ridges, grooves, etc. disposed on each of their respective surfaces, even though each such feature is not necessarily fully illustrated and described for this particular jaw assembly. For example, one jaw 535C can be shorter than the other two jaws 535A, 535B. The other two jaws 535A, 535B can have the same lengths and/or lengths that are generally longer than the shorter jaw 535C. Jaw assemblies that include three jaws, one of which is shorter than the other two jaws, can be beneficial because they can allow the operator of the medical device to use the two longer jaws for making incisions or cuts *(e.g.,* otomy creation) and/or for making fine dissection, while maintaining the tissue in its position and grasping the tissue in place using the shorter third jaw. A person skilled in the art will recognize other functions that can be performed by an arrangement of three or more jaws.

Regardless of the number of jaws included in the jaw assembly 535, the jaws can be arranged to operate fully in concert *(e.g.,* in agreement or in coordination with one another), partially in concert, or completely independent of one another. For example, as shown in FIG. 5B, the operator of the medical device can use the jaw assembly 535 in its closed position to create cuts or incisions in the tissue. Further, by incorporating ridges into an outer surface thereof (not shown), such as the ridges 440 of the jaw assembly 435, abrasions can be formed by dragging the jaw assembly 535 against tissue. Since the jaws can operate independently of one another, depending on the application, the operator can use the medical device in a partially open position (FIG. 5C), for example with one jaw 535B partially opened to initiate tissue grasping. Depending on the application, while the operator is working on the tissue, one or more of the jaws can remain stationary.

Alternatively or additionally, one or more of the jaws can be fully or partially opened. For example, the operator can choose to partially or fully open a jaw 535A to assist in making an incision larger and/or partially or fully open yet another jaw 535C to assist in maintaining the tissue in place and/or to assist with grasping of the tissue. Alternatively or additionally, one or more of the jaws 535A, 535B, 535C can remain stationary during the operation of the device. Notably, while in the illustrated embodiment or a partially opened configuration it is shown that the jaws 535A and 535C remain substantially in contact with one another while the jaw 535B pivots with respect to those two jaws, the jaws 535B and 535C can remain substantially in contact with one another while the jaw 535A pivots with respect to those two jaws.

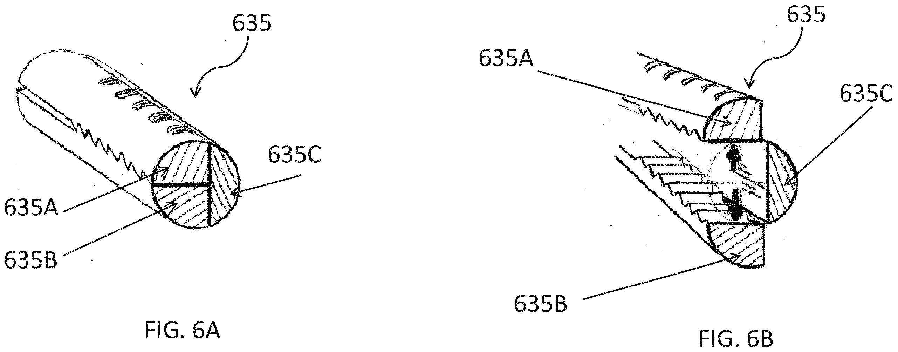

In some embodiments, the jaws 535A, 535B, 535C can be arranged such that they open at different times (instances) during the operation of the device. Accordingly, depending on the application at hand, each of the jaws 535A, 535B, 535C included in the jaw assembly 535 can be opened (partially or fully) at any time while the operator is working with the device. Specifically, the jaws can be configured such that one or more jaws partially or fully open after a certain predetermined amount of time since opening and/or operation of another jaw (or other jaws) has passed. For example, if a first jaw 535A opens at time, *m*, the second jaw 535B can be arranged such that it opens after a predetermined amount of time, *n,* has lapsed (opens at *m* + *n*)*.* The control of the timing of the opening of the jaws can be automated by coupling one or more automatic controllers with the medical device, or by using mechanical components designed to delay the actuation of one component with respect to another, as would be understood by a person having skill in the art and also described in further detail below with respect to a jaw assembly 635 in FIGS. 6A-6C. Further, one or more automatic or manual controllers (not shown) can be coupled with the medical device to measure and control the rotation angles at which each jaw opens. Further, the jaws can be arranged such that they open at different rates or speeds. By way of non-limiting example, some medical devices can be configured such that after the first and second jaws 535A, 535B have opened, the third jaw 535C can open at a faster rate than the first and second jaws. FIGS. 6A-6C provide a detailed illustration of one exemplary, non-limiting way by which different jaws can be opened and/or closed at different times and/or rates.

FIG. 6A illustrates a jaw assembly 635 having three complementary jaws 635A, 635B, 635C disposed in a closed configuration. In this jaw assembly, the three jaws are of an equal length, although as explained above, in other jaw assemblies the jaws can have different lengths from each other. Two of the jaws 635A and 635B, are nearly mirror images of each other, having complementary ridges and grooves formed on their opposed tissue-facing surfaces and a plurality of grooves formed on an external surface (not visible for jaw 635B). The third jaw 635C fits with the other two jaws to form a generally cylindrical shape, and has an inward facing flat surface that is complementary to flat surfaces of the first and second jaws 635A, 635B. In other jaw assemblies, the jaws can be configured in a manner similar to the configurations described with respect to the other disclosed jaw assemblies provided for herein (*e.g*., jaw assemblies 435 and 535), otherwise derivable therefrom, or otherwise known to those skilled in the art.

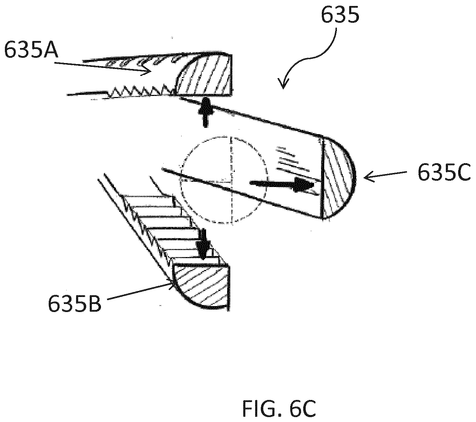

FIG. 6B illustrates the jaw assembly 635 moving into a partially open position. As shown, the first and second jaws 635A, 635B extend directly away from each other, while the third jaw 635C remains stationary. As noted above, depending on the application, an operator may choose to allow one or more jaws to open (possibly to create an incision and/or hold and grasp the tissue) but maintain the remaining jaw(s) in a stationary position (*e.g*., to prevent an incision from getting too large and/or to use the third jaw in maintaining and grasping the tissue). As shown in FIG. 6C, in which the jaw assembly 635 is in the fully open position, each of the three jaws 635A, 635B, and 635C has now advanced radially outward from their locations in the closed position.

As discussed above, the third jaw 635C can advance at a faster rate to "catch-up" to the other jaws 635A, 635B, or the other jaws can be configured to stop at a certain point, after which the third jaw 635C can continue to advance radially outward to the fully open position. The timing and rate at which the jaws move can be programmed or controlled into the instrument itself, for instance by providing racks, pinions, gears, and the like having different sizes and configurations, and/or the can be manually controlled or dictated by an operator. Generally, each of the jaws can be configured to operate independently of one another, or together in some combination if desired. A person skilled in the art will recognize that although the fully closed and open positions illustrated show the jaws 635A, 635B, 635C generally each moving a similar distance, in other configurations some jaws may move more or less than others and at faster or slower speeds, depending, at least in part, on the design of the device, the desired effect by the operator, and the type of procedure being performed.

As noted above, the jaws in the jaw assembly 635 can be arranged to assume various shapes, lengths, width, geometries, thicknesses, and ridge configurations across their surfaces. Further, as noted, one or more of the features (or all of the features) included in each jaw can be independent from the features included on other jaws and/or be complimentary such that it allows at least one portion of the jaw to mate with at least one portion of another jaw.

In use, the jaw assembly 435 (or the jaw assemblies 535 and 635 to the extent they incorporate particular features described above with respect to the jaw assembly, *e.g*., shapes and configurations of the jaws, ridges 440, bulbous tips, etc.) can be used in each of the three dissection modes discussed above. For instance, when operating on a patient, the operator can drag the outer surface of the jaws, *i.e.,* the ridges and grooves, against tissue to form one or more abrasions in the tissue. Further, the operator can also move or pivot the jaws with respect to each other to spread tissue apart. This can be done after using the distal end of the jaws, or other portion thereof, to form an initial incision in the jaws, or just by inserting the jaws into an existing opening (whether naturally occurring or otherwise formed) and spreading the jaws to open the tissue. As discussed above, in some instances, for example in configurations in which there are three jaws, the timing and rate at which the jaws spread can be varied and thus not uniform for each of the jaws. Still further, the operator can grasp tissue using the first and second jaws, for instance by clamping down on tissue disposed between the jaws. Beneficially, each of these actions can be performed by the same end effector because of the various features provided for in the design.

During the course of performing the procedure, an operator can identify which features of the jaw assemblies to use to effect different results. This can include selectively using only a portion of an outer surface of one or more of the jaws to perform one or more of the desired functions. For example, based on the particular tissue being operated on, an operator can identify a particular portion of the outer surface of one or more of the jaws to perform a particular step, *e.g.,* dragging to form abrasion(s) or moving to spread tissue apart. By way of non-limiting example, an operator may prefer to use the thicker, wider, bulbous tip to perform the dragging step rather than the smaller, thinner, intermediate portion of the same jaw. By way of further non-limiting example, an operator may prefer a particular curvature of a portion of an inner or outer surface of one or more of the jaws to perform the moving/spreading step. A person skilled in the art, in view of the present disclosures, will recognize a variety of different steps that can be performed to achieve certain desirable results based on the jaw configurations provided for herein or otherwise derivable from the present disclosures.

A person skilled in the art will appreciate that the present disclosure has application in conventional endoscopic and open surgical instrumentation as well application in roboticassisted surgery.

The devices disclosed herein can be designed to be disposed of after a single use, or they can be designed to be used multiple times. In either case, however, the device can be reconditioned for reuse after at least one use. Reconditioning can include any combination of the steps of disassembly of the device, followed by cleaning or replacement of particular pieces, and subsequent reassembly. In particular, the device can be disassembled, and any number of the particular pieces or parts of the device can be selectively replaced or removed in any combination. Upon cleaning and/or replacement of particular parts, the device can be reassembled for subsequent use either at a reconditioning facility, or by a surgical team immediately prior to a surgical procedure. Those skilled in the art will appreciate that reconditioning of a device can utilize a variety of techniques for disassembly, cleaning/replacement, and reassembly.

Preferably, the devices described herein will be processed before surgery. First, a new or used instrument is obtained and if necessary cleaned. The instrument can then be sterilized. In one sterilization technique, the instrument is placed in a closed and sealed container, such as a plastic or TYVEK^{®} bag. The container and its contents are then placed in a field of radiation that can penetrate the container, such as gamma radiation, x-rays, or high-energy electrons. The radiation kills bacteria on the instrument and in the container. The sterilized instrument can then be stored in the sterile container. The sealed container keeps the instrument sterile until it is opened in the medical facility.

It is preferred that device is sterilized. This can be done by any number of ways known to those skilled in the art including beta or gamma radiation, ethylene oxide, steam.

One skilled in the art will appreciate further features and advantages of the invention based on the above-described embodiments. Accordingly, the invention is not to be limited by what has been particularly shown and described, except as indicated by the appended claims.

## Claims

1. Jaws (435) for a surgical device comprising:
a first jaw (435A) having a length, a width, and a thickness, the length being greater than the width, and the first jaw including a tissue-facing surface (435Aint) and an outer surface (435A_{Ext}), with the thickness being part of the outer surface;
a second jaw (435B), opposed to and pivotally connected to the first jaw, the second jaw having a length, a width, and a thickness, the length being greater than the width, and the second jaw including a tissue-facing surface (435B_{Int}) and an outer surface (435B_{Ext}), the tissue-facing surface of the second jaw facing the tissue-facing surface of the first jaw, and the thickness of the second jaw being part of the outer surface of the second jaw,
wherein the tissue-facing surfaces of the first and second jaws each include a plurality of ridges (440), the ridges extending along a substantial portion of the width of the respective jaws, and
wherein the outer surfaces of the first and second jaws each include a plurality of ridges (440), the ridges extending along a substantial portion of the width of the respective jaws, and the plurality of ridges of at least one of the outer surfaces of the first and second jaws extending onto a portion of the outer surface that defines the thickness for that jaw;
**characterized in that** a distal end of one of the first and second jaws includes a tissue-grasping groove (460) formed in the tissue-facing surface, the groove being substantially perpendicular to the plurality of ridges of the tissue-facing surface, and a distal end of the other of the first and second jaws includes a tissue-grasping tooth (462) disposed on the tissue-facing surface, the tooth being substantially perpendicular to the plurality of ridges of the tissue-facing surface and being complimentary to the tissue-grasping groove of the other jaw.

2. The device of claim 1, wherein one or more ridges of the plurality of ridges on the outer surface of the first jaw is aligned with one or more ridges of the plurality of ridges on the outer surface of the second jaw such that when the first and second jaws are in a closed configuration, the aligned one or more ridges of the first and second jaws form one or more continuous ridges across the first and second jaws.

3. The device of claim 1 or claim 2, wherein a portion of the outer surface of the first or second jaw that is opposed to the portion of the outer surface that defines the thickness for that jaw on which the plurality of ridges extend does not include the plurality of ridges formed thereon.

4. The device of any preceding claim, wherein a first side surface of the first jaw has a radius of curvature (435A_{C3}, 435A_{C4}) that is different from a radius of curvature (435A_{C5}, 435A_{C6}, 435A_{C7}) of an opposed second side surface of the first jaw when the jaw is viewed from a top view, looking directly onto the outer surface of the first jaw from above, the first and second side surfaces being part of the outer surface of the first jaw.

5. The device of claim 4,
wherein the radius of curvature of the first side surface is substantially the same along a majority of the length of the first jaw, and
wherein the radius of curvature of the second side surface changes along the length of the second jaw.

6. The device of claim 4 or claim 5, wherein a distal end of the second side surface is convex and an intermediate portion of the second side surface is concave.

7. The device of any preceding claim, wherein a width at a distal end of the first jaw is greater than a width at an intermediate portion along the length of the first jaw.

8. The device of any preceding claim,
wherein the first jaw has a thickness, the thickness being part of the outer surface of the first jaw, and
wherein a thickness of the first jaw at a distal end of the first jaw is greater than a thickness of the first jaw at an intermediate portion along the length of the first jaw.

9. The device of any preceding claim, wherein the first jaw has a distal end that is bulbous.

10. An endoscopic device, comprising:
an actuator (106); and
the device of claim 1 operably coupled to the actuator such that operation of the actuator is configured to open and close the opposed jaws.

11. The endoscopic device of claim 10, wherein, when viewing the jaw assembly from a top view, looking directly onto the top surface of one of the jaws of the jaw assembly, a first side surface of the distal end of the jaw assembly is concave while an opposed second side surface of the distal end of the jaw assembly is convex.

## Patentansprüche

1. Backen (435) für eine chirurgische Vorrichtung, umfassend:
eine erste Backe (435A) mit einer Länge, einer Breite und einer Dicke, wobei die Länge größer als die Breite ist und die erste Backe eine Gewebe zugewandte Fläche (435A_{Int}) und eine Außenfläche (435A_{Ext}) aufweist, wobei die Dicke Teil der Außenfläche ist,
eine zweite Backe (435B), die der ersten Backe gegenüberliegt und schwenkbar mit dieser verbunden ist, wobei die zweite Backe eine Länge, eine Breite und eine Dicke hat, wobei die Länge größer als die Breite ist und die zweite Backe eine Gewebe zugewandte Fläche (435B_{Int}) und eine Außenfläche (435B_{Ext}) aufweist, wobei die Gewebe zugewandte Fläche der zweiten Backe der Gewebe zugewandten Fläche der ersten Backe zugewandt ist und die Dicke der zweiten Backe Teil der Außenfläche der zweiten Backe ist,
wobei die Gewebe zugewandten Flächen der ersten und der zweiten Backe jeweils eine Vielzahl von Erhöhungen (440) aufweisen, wobei sich die Erhöhungen entlang eines wesentlichen Abschnitts der Breite der jeweiligen Backen erstrecken, und
wobei die Außenflächen der ersten und der zweiten Backe jeweils eine Vielzahl von Erhöhungen (440) aufweisen, wobei sich die Erhöhungen entlang eines wesentlichen Abschnitts der Breite der jeweiligen Backen erstrecken und sich die Vielzahl von Erhöhungen mindestens einer der Außenflächen der ersten und der zweiten Backe auf einen Abschnitt der Außenfläche, der die Dicke dieser Backe definiert, erstrecken,
**dadurch gekennzeichnet, dass** ein distales Ende der ersten oder der zweiten Backe eine Gewebe ergreifende Nut (460) aufweist, die in der Gewebe zugewandten Fläche ausgebildet ist, wobei die Nut im Wesentlichen senkrecht zu der Vielzahl von Erhöhungen der Gewebe zugewandten Fläche verläuft, und ein distales Ende der anderen der ersten oder der zweiten Backe einen Gewebe ergreifende Zahn (462) aufweist, der an der Gewebe zugewandten Fläche angeordnet ist, wobei der Zahn im Wesentlichen senkrecht zu der Vielzahl von Erhöhungen der Gewebe zugewandten Fläche verläuft und zu der Gewebe ergreifenden Nut der anderen Backe komplementär ist.

2. Vorrichtung nach Anspruch 1, wobei eine oder mehrere Erhöhungen der Vielzahl von Erhöhungen auf der Außenfläche der ersten Backe auf eine oder mehrere Erhöhungen der Vielzahl von Erhöhungen auf der Außenfläche der zweiten Backe ausgerichtet sind, so dass die ausgerichteten eine oder mehreren Erhöhungen der ersten und der zweiten Backe eine oder mehrere durchgehende Erhöhungen über die erste und die zweite Backe bilden, wenn die erste und die zweite Backe in einer geschlossenen Konfiguration sind.

3. Vorrichtung nach Anspruch 1 oder Anspruch 2, wobei ein Abschnitt der Außenfläche der ersten oder der zweiten Backe, der dem Abschnitt der Außenfläche gegenüberliegt, der die Dicke für die Backe definiert, an der sich die Vielzahl von Erhöhungen erstrecken, die Vielzahl von daran ausgebildeten Erhöhungen nicht aufweist.

4. Vorrichtung nach einem der vorhergehenden Ansprüche, wobei eine erste Seitenfläche der ersten Backe einen Krümmungsradius (435A_{C3}, 435A_{C4}) hat, der von einem Krümmungsradius (435A_{C5},435A_{C6}, 435A_{C7}) einer gegenüberliegenden Seitenfläche der ersten Backe verschieden ist, wenn die Backe von oben betrachtet wird und man direkt auf die Außenfläche der ersten Backe von oben schaut, wobei die erste und die zweite Seitenfläche Teil der Außenfläche der ersten Backe sind.

5. Vorrichtung nach Anspruch 4,
wobei der Krümmungsradius der ersten Seitenfläche entlang eines Großteils der Länge der ersten Backe im Wesentlichen derselbe ist, und
wobei sich der Krümmungsradius der zweiten Seitenfläche entlang der Länge der zweiten Backe ändert.

6. Vorrichtung nach Anspruch 4 oder Anspruch 5, wobei ein distales Ende der zweiten Seitenfläche konvex ist und ein Zwischenabschnitt der zweiten Seitenfläche konkav ist.

7. Vorrichtung nach einem der vorhergehenden Ansprüche, wobei eine Breite an einem distalen Ende der ersten Backe größer als eine Breite an einem Zwischenabschnitt entlang der Länge der ersten Backe ist.

8. Vorrichtung nach einem der vorhergehenden Ansprüche,
wobei die erste Backe eine Dicke hat, wobei die Dicke Teil der Außenfläche der ersten Backe ist und
wobei eine Dicke der ersten Backe an einem distalen Ende der ersten Backe größer als eine Dicke der ersten Backe an einem Zwischenabschnitt entlang der Länge der ersten Backe ist.

9. Vorrichtung nach einem der vorhergehenden Ansprüche, wobei die erste Backe ein distales Ende hat, das knollenförmig ist.

10. Endoskopvorrichtung, umfassend:
einen Aktuator (106) und
die Vorrichtung nach Anspruch 1, die so an den Aktuator wirkgekoppelt ist, dass die Betätigung des Aktuators zum Öffnen und Schließen der gegenüberliegenden Backen ausgestaltet ist.

11. Endoskopvorrichtung nach Anspruch 10, wobei, wenn die Backenanordnung von oben betrachtet wird und man direkt auf die obere Fläche einer der Backen der Backenanordnung schaut, eine erste Seitenfläche des distalen Endes der Backenanordnung konkav ist, während eine gegenüberliegende zweite Seitenfläche des distalen Endes der Backenanordnung konvex ist.

## Revendications

1. Mâchoires (435) pour un dispositif chirurgical comprenant :
une première mâchoire (435A) ayant une longueur, une largeur et une épaisseur, la longueur étant supérieure à la largeur, et la première mâchoire comprenant une surface faisant face au tissu (435A_{Int}) et une surface extérieure (435A_{Ext}), l'épaisseur faisant partie de la surface extérieure ;
une seconde mâchoire (435B), opposée et reliée de manière pivotante à la première mâchoire, la seconde mâchoire ayant une longueur, une largeur et une épaisseur, la longueur étant supérieure à la largeur, et la seconde mâchoire comprenant une surface faisant face au tissu (435B_{Int}) et une surface extérieure (435B_{Ext}), la surface faisant face au tissu de la seconde mâchoire faisant face à la surface faisant face au tissu de la première mâchoire, et l'épaisseur de la seconde mâchoire faisant partie de la surface extérieure de la seconde mâchoire,
les surfaces faisant face au tissu des première et seconde mâchoires comprenant chacune une pluralité de nervures (440), les nervures s'étendant le long d'une partie substantielle de la largeur des mâchoires respectives, et
les surfaces extérieures des première et seconde mâchoires comprenant chacune une pluralité de nervures (440), les nervures s'étendant le long d'une partie substantielle de la largeur des mâchoires respectives, et la pluralité de nervures d'au moins une des surfaces extérieures des première et seconde mâchoires s'étendant sur une partie de la surface extérieure qui définit l'épaisseur pour cette mâchoire ;
**caractérisées en ce qu'**une extrémité distale de l'une des première et seconde mâchoires comprend une rainure de préhension de tissu (460) formée dans la surface faisant face au tissu, la rainure étant sensiblement perpendiculaire à la pluralité de nervures de la surface faisant face au tissu, et une extrémité distale de l'autre des première et seconde mâchoires comprend une dent de préhension de tissu (462) disposée sur la surface faisant face au tissu, la dent étant sensiblement perpendiculaire à la pluralité de nervures de la surface faisant face au tissu et étant complémentaire de la rainure de préhension de tissu de l'autre mâchoire.

2. Dispositif selon la revendication 1, une ou plusieurs nervures de la pluralité de nervures sur la surface extérieure de la première mâchoire étant alignées avec une ou plusieurs nervures de la pluralité de nervures sur la surface extérieure de la seconde mâchoire de telle sorte que lorsque les première et seconde mâchoires sont dans une configuration fermée, la ou les nervures alignées des première et seconde mâchoires forment une ou plusieurs nervures continues à travers les première et seconde mâchoires.

3. Dispositif selon la revendication 1 ou selon la revendication 2, une partie de la surface extérieure de la première ou de la seconde mâchoire qui est opposée à la partie de la surface extérieure qui définit l'épaisseur pour cette mâchoire sur laquelle la pluralité de nervures s'étend ne comprenant pas la pluralité de nervures formées sur celle-ci.

4. Dispositif selon l'une quelconque des revendications précédentes, une première surface latérale de la première mâchoire ayant un rayon de courbure (435A_{C3}, 435A_{C4}) qui est différent d'un rayon de courbure (435A_{C5},435A_{C6}, 435A_{C7}) d'une seconde surface latérale opposée de la première mâchoire lorsque la mâchoire est vue depuis une vue de dessus, en regardant directement sur la surface extérieure de la première mâchoire depuis le dessus, les première et seconde surfaces latérales faisant partie de la surface extérieure de la première mâchoire.

5. Dispositif selon la revendication 4,
le rayon de courbure de la première surface latérale étant sensiblement le même sur une majorité de la longueur de la première mâchoire, et
le rayon de courbure de la seconde surface latérale changeant le long de la longueur de la seconde mâchoire.

6. Dispositif selon la revendication 4 ou selon la revendication 5, une extrémité distale de la seconde surface latérale étant convexe et une partie intermédiaire de la seconde surface latérale étant concave.

7. Dispositif selon l'une quelconque des revendications précédentes, une largeur à une extrémité distale de la première mâchoire étant supérieure à une largeur à une partie intermédiaire sur la longueur de la première mâchoire.

8. Dispositif selon l'une quelconque des revendications précédentes,
la première mâchoire ayant une épaisseur, l'épaisseur faisant partie de la surface extérieure de la première mâchoire, et
une épaisseur de la première mâchoire à une extrémité distale de la première mâchoire étant supérieure à une épaisseur de la première mâchoire à une partie intermédiaire le long de la longueur de la première mâchoire.

9. Dispositif selon l'une quelconque des revendications précédentes, la première mâchoire ayant une extrémité distale qui est bulbeuse.

10. Dispositif endoscopique, comprenant :
un actionneur (106) ; et
le dispositif selon la revendication 1, couplé de manière fonctionnelle à l'actionneur de telle sorte que le fonctionnement de l'actionneur est configuré pour ouvrir et fermer les mâchoires opposées.

11. Dispositif endoscopique selon la revendication 10, lors de l'observation de l'ensemble de mâchoires depuis une vue de dessus, en regardant directement sur la surface supérieure de l'une des mâchoires de l'ensemble de mâchoires, une première surface latérale de l'extrémité distale de l'ensemble de mâchoires étant concave tandis qu'une seconde surface latérale opposée de l'extrémité distale de l'ensemble de mâchoires est convexe.
